# EUROPEAN PATENT SPECIFICATION

(11) **EP 0 587 738 B1**
(45) Date of publication and mention of the grant of the patent: **23.08.2000**
(21) Application number: 92912929.4
(22) Date of filing: 05.06.1992
(51) Int. Cl.: A61K 47/48

(54) **TARGETED DELIVERY OF GENES ENCODING SECRETORY PROTEINS**
ZIELGERICHTETE FREISETZUNG VON GENEN, DIE SEKRETORISCHE PROTEINE KODIEREN
APPORT CIBLE DE GENES CODANT DES PROTEINES SECRETOIRES

(30) Priority: 05.06.1991 US 710558
(43) Date of publication of application: 23.03.1994
(73) Proprietor: UNIVERSITY OF CONNECTICUT, Storrs, Connecticut 06269 (US); THE BOARD OF REGENTS ACTING FOR AND ON BEHALF OF THE UNIVERSITY OF MICHIGAN, Ann Arbor, MI 48109-1248 (US)
(72) Inventor: WU, George Y., Bloomfield, CT 06002 (US); WILSON, James M., Ann Arbor, MI 48103 (US); WU, Catherine H., Avon CT 06001-3825 (US)
(74) Representative: UEXKÜLL & STOLBERG
(86) International application number: US9204565
(87) International publication number: WO9222635

(56) References cited:
- WO-A-90/12096
- JOURNAL OF BIOLOGICAL CHEMISTRY, Vol. 264, No. 29, 15 October 1989, Baltimore, USA, pages 16985-16987, WU et al., "Targeting Genes: Delivery and Persistent Expression of a Foreign Gene Driven by Mammalian Regulatory Elements In Vivo".
- JOURNAL OF BIOLOGICAL CHEMISTRY, Vol. 264, No. 21, 25 July 1989, Baltimore, USA, pages 12126-12129, KANEDA et al., "Introduction And Expression of the Human Insulin Gene in Adult Rat Liver".
- JOURNAL OF BIOLOGICAL CHEMISTRY, Vol. 266, No. 6, 25 February 1991, Baltimore, USA, pages 3361-3364, KATO et al., "Expression of Hepatitis B Virus Surface Antigen in Adult Rat Liver".
- PROC. NATL. ACAD. SCI. U.S.A., Vol. 87, April 1990, Washington D.C., USA, pages 2652-2656, SHALABY et al., "Exon Skipping During Splicing of Albumin mRNA Precursors in Nagase Analbuminemic Rats".
- JOURNAL OF BIOLOGICAL CHEMISTRY, Vol. 266, No. 22, 5 August 1991, Baltimore, USA, pages 14338-14342, WU et al., "Receptor-Mediated Gene Delivery In Vivo".

## Description

### Government Support

The work leading to this invention was supported, in part, by research grants from the United States government.

### Background of the Invention

Many secreted proteins have been studied in a variety of cell types and all of them follow a similar pathway of secretion. The protein is synthesized in the cell cytosol by the process of translation which is performed by ribosomes located on the cytosolic side of the endoplasmic reticulum. The protein is then transported into the endoplasmic reticulum - Golgi apparatus for ultimate secretion from the cell.

The secretion of a protein is directed by a signal peptide which is usually located at the amino-terminus of the protein. This peptide is removed as the protein passes from the ribosome into the endoplasmic reticulum and therefore it does not appear in the mature, secreted protein.

Secretory proteins such as hormones or enzymes are involved in many biological processes. Severe abnormalities can result from the absence or insufficient secretion of such proteins. Methods for alleviating or correcting defects in the production of secretory proteins are needed.

### Summary of the Invention

This invention pertains to a soluble molecular complex for targeting a gene encoding a secretory protein to a specific cell in vivo and obtaining secretion of the protein by the targeted cell. The molecular complex comprises an expressible gene encoding a desired secretory protein complexed to a carrier which is a conjugate of a cell-specific binding agent and a gene-binding agent. The cell-specific binding agent is specific for a cellular surface structure, typically a receptor, which mediates internalization of bound ligands by endocytosis, such as the asialoglycoprotein receptor of hepatocytes. The cell-specific binding agent can be a natural or synthetic ligand (for example, a protein, polypeptide, glycoprotein, etc.) or it can be an antibody, or an analogue thereof, which specifically binds a cellular surface structure which then mediates internalization of the bound complex. The gene-binding component of the conjugate is a compound such as a polycation which stably complexes the gene under extracellular conditions and releases the gene under intracellular conditions so that it can function within the cell.

The complex of the gene and the carrier is stable and soluble in physiological fluids. It can be administered in vivo where it is selectively taken up by the target cell via the surface-structure-mediated endocytotic pathway. The incorporated gene is expressed and the gene-encoded product is processed and secreted by the transfected cell.

The soluble molecular complex of this invention can be used to specifically transfect cells in vivo to provide for expression and secretion of a desired protein. This selective transfection is useful for gene therapy and in other applications which require selective genetic alteration of cells to yield a secretable protein product. In gene therapy, a normal gene can be targeted to a specific cell to correct or alleviate an inherited or acquired abnormality involving a secretory protein, such as blood-coagulant deficiency, caused by a defect in a corresponding endogenous gene.

### Brief Description Of The Figures

Figure 1 shows the structure of the plasmid vectors palb³ and palb², each of which contains a gene encoding the secretory protein albumin. Palb³ contains the structural gene for human serum albumin driven by the rat albumin promoter and the mouse albumin enhancer regions. Palb² is a control vector which lacks the mouse albumin enhancer sequence which is necessary for high levels of expression of the albumin gene.
Figure 2 shows Southern blots which indicate the presence and abundance of plasmid DNA targeted by the method of this invention to liver cells of rats.
Figure 3 shows dot blots of hepatic RNA which indicate transcription of the vector-derived serum albumin gene by the liver cells.
Figure 4 shows RNase protection analysis which confirms the presence of vector-derived human serum albumin mRNA in the liver cells.
Figure 5 is a Western blot which confirms the presence of human serum albumin in rat serum.
Figure 6 shows levels of circulating human albumin in rat serum as a function of time after injection with palb³ DNA complex and partial hepatectomy.

### Detailed Description of the Invention

A soluble, targetable molecular complex is used to selectively deliver a gene encoding a secretory protein to a target cell or tissue in vivo. The molecular complex comprises the gene to be delivered complexed to a carrier made up of a binding agent specific for the target cell and a gene-binding agent. The complex is selectively taken up by the target cell and the gene product is expressed and secreted.

The gene, generally in the form of DNA, encodes the desired secretory protein (or glycoprotein). Typically, the gene comprises a structural gene encoding the desired protein in a form suitable for processing and secretion by the target cell. For example, the gene encodes appropriate signal sequences which provide for cellular secretion of the product. The signal sequence may be the natural sequence of the protein or exogenous sequences. The structural gene is linked to appropriate genetic regulatory elements required for expression of the gene product by the target cell. These include a promoter and optionally an enhancer element operable in the target cell. The gene can be contained in an expression vector such as a plasmid or a transposable genetic element along with the genetic regulatory elements necessary for expression of the gene and secretion of the gene-encoded product.

The carrier component of the complex is a conjugate of a cell-specific binding agent and a gene-binding agent. The cell-specific binding agent specifically binds a cellular surface structure which mediates its internalization by, for example, the process of endocytosis. The surface structure can be a protein, polypeptide, carbohydrate, lipid or combination thereof. It is typically a surface receptor which mediates endocytosis of a ligand. Thus, the binding agent can be a natural or synthetic ligand which binds the receptor. The ligand can be a protein, polypeptide, glycoprotein or glycopeptide which has functional groups that are exposed sufficiently to be recognized by the cell surface structure. It can also be a component of a biological organism such as a virus, cells (e.g., mammalian, bacterial, protozoan) or artificial carriers such as liposomes.

The binding agent can also be an antibody, or an analogue of an antibody such as a single chain antibody, which binds the cell surface structure.

Ligands useful in forming the carrier will vary according to the particular cell to be targeted. For targeting hepatocytes, glycoproteins having exposed terminal carbohydrate groups such as asialoglycoprotein (galactose-terminal) can be used, although other ligands such as polypeptide hormones may also be employed. Examples of asialoglycoproteins include asialoorosomucoid, asialofetuin and desialylated vesicular stomatitis virus. Such ligands can be formed by chemical or enzymatic desialylation of glycoproteins that possess terminal sialic acid and penultimate galactose residues. Alternatively, asialoglycoprotein ligands can be formed by coupling galactose terminal carbohydrates such as lactose or arabinogalactan to non-galactose bearing proteins by reductive lactosamination.

For targeting the molecular complex to other cell surface receptors, other types of ligands can be used, such as mannose for macrophages (lymphoma), mannose-6-phosphate glycoproteins for fibroblasts (fibrosarcoma), intrinsic factor-vitamin B12 for enterocytes and insulin for fat cells. Alternatively, the cell-specific binding agent can be a receptor or receptor-like molecule, such as an antibody which binds a ligand (e.g., antigen) on the cell surface. Such antibodies can be produced by standard procedures.

The gene-binding agent complexes the gene to be delivered. Complexation with the gene must be sufficiently stable in vivo to prevent significant uncoupling of the gene extracellularly prior to internalization by the target cell. However, the complex is cleavable under appropriate conditions within the cell so that the gene is released in functional form. For example, the complex can be labile in the acidic and enzyme rich environment of lysosomes. A noncovalent bond based on electrostatic attraction between the gene-binding agent and the expressible gene provides extracellular stability and is releasable under intracellular conditions.

Preferred gene-binding agents are polycations that bind negatively charged polynucleotides. These positively charged materials can bind noncovalently with the gene to form a soluble, targetable molecular complex which is stable extracellularly but releasable intracellularly. Suitable polycations are polylysine, polyarginine, polyornithine, basic proteins such as histones, avidin, protamines and the like. A preferred polycation is polylysine (e.g., ranging from 3,800 to 60,000 daltons). Other noncovalent bonds that can be used to releasably link the expressible gene include hydrogen bonding, hydrophobic bonding, electrostatic bonding alone or in combination such as, anti-polynucleotide antibodies bound to polynucleotide, and strepavidin or avidin binding to polynucleotide containing biotinylated nucleotides.

The linkage reaction between the cell-specific binding agent and the gene-binding agent can be optimized for the particular cell-specific binding agent and gene-binding agent used to form the carrier. Reaction conditions can be designed to maximize linkage formation but to minimize the formation of aggregates of the carrier components. The optimal ratio of cell-specific binding agent to gene-binding agent can be determined empirically. When polycations are used, the molar ratio of the components will vary with the size of the polycation and the size of the gene. In general, this ratio ranges from about 10:1 to 1:1, preferably about 5:1. Uncoupled components and aggregates can be separated from the carrier by molecular sieve or ion exchange chromatography (e.g., Aquapore™ cation exchange, Rainan).

The gene encoding the secretory protein can be complexed to the carrier by a stepwise dialysis procedure. In a preferred method, for use with carriers made of polycations such as polylysine, the dialysis procedure begins with a 2M NaCl dialyzate and ends with a .15M NaCl solution. The gradually decreasing NaCl concentration results in binding of the gene to the carrier. In some instances, particularly when concentrations of the gene and carrier are low, dialysis may not be necessary; the gene and carrier are simply mixed and incubated.

The molecular complex can contain more than one copy of the same gene or one or more different genes. Preferably, the ratio of gene to the carrier is from about 1:5 to 5:1, preferably about 1:2.

The molecular complex of this invention can be administered parenterally. Preferably, it is injected intravenously. The complex is administered in solution in a physiologically acceptable vehicle.

Cells can be transfected in vivo for transient expression and secretion of the gene product. For prolonged expression and secretion, the gene can be administered repeatedly. Alternatively, the transfected target cell can be stimulated to replicate by surgical or pharmacological means to prolong expression of the incorporated gene. See, for example, U.S. Patent Application Serial No. 588,013, filed September 25, 1990, the teachings of which are incorporated by reference herein.

The method of this invention can be used in gene therapy to selectively deliver a gene encoding a secretory protein to a target cell in vivo for expression and secretion of the gene-encoded product by the cell. For example, a normal gene can be targeted to a specific cell to correct or alleviate a metabolic or genetic abnormality caused by an inherited or acquired defect in a corresponding endogenous gene.

The molecular complex of this invention is adaptable for delivery of a wide range of genes to a specific cell or tissue. Preferably, the complex is targeted to the liver by exploiting the hepatic asialoglycoprotein receptor system which allows for in vivo transfection of hepatocytes by the process of receptor-mediated endocytosis. The liver has the highest rate of protein synthesis per gram of tissue. Thus, the molecular complex of this invention can be used to specifically target the liver as a site for high efficiency production of a therapeutic secretory protein to treat hepatic abnormalities or abnormalities in other tissues.

The method of the invention can be used to treat inherited states of blood coagulant-deficiency. These include deficiencies in any of the clotting factors II-XIII. Factors V, VII, IX, X or XI are normally made in the liver. Factor VIII is normally made in endothelial cells and in liver parenchymal cells. In a preferred embodiment, the gene encoding the clotting factor is complexed to a conjugate of an asialoglycoprotein and a polycation. The resulting soluble complex is administered parenterally to target liver cells of the individual afflicted with the deficiency in amounts sufficient to selectively transfect the cells and to provide sufficient secretion of the factor to attain circulating levels for effective clotting activity.

This invention is illustrated further by the following Exemplification.

### EXEMPLIFICATION

### Example 1

An asialoglycoprotein-polycation conjugate consisting of asialoorosomucoid coupled to poly-L-lysine, was used to form a soluble DNA complex capable of specifically targeting hepatocytes via asialoglycoprotein receptors present on these cells. The DNA comprised a plasmid, palb³, containing the structural gene for human serum albumin driven by mouse albumin enhancer-rat albumin promoter elements.

### Formation of the Molecular Complex

### Animals

An animal model of a genetic metabolic disorder, the Nagase analbuminemic rat, was selected. This strain possesses a defect in splicing of mRNA of serum albumin resulting in virtually undetectable levels of circulating serum albumin (Nagase, S. et al. Science 205:590-591 (1979); Shalaby, F. and Shafritz, D.A. Proc. Natl. Acad. Sci. (USA) 87:2652-26756 (1990)). Male, 200-250 g, Nagase analbuminemic rats were kindly provided by Dr. Jayanta Roy Chowdhury (Albert Einstein College of Medicine, Bronx, New York) and maintained in light-dark cycles and fed ad lib.

### Expression Vectors Containing the Human Serum Albumin Gene

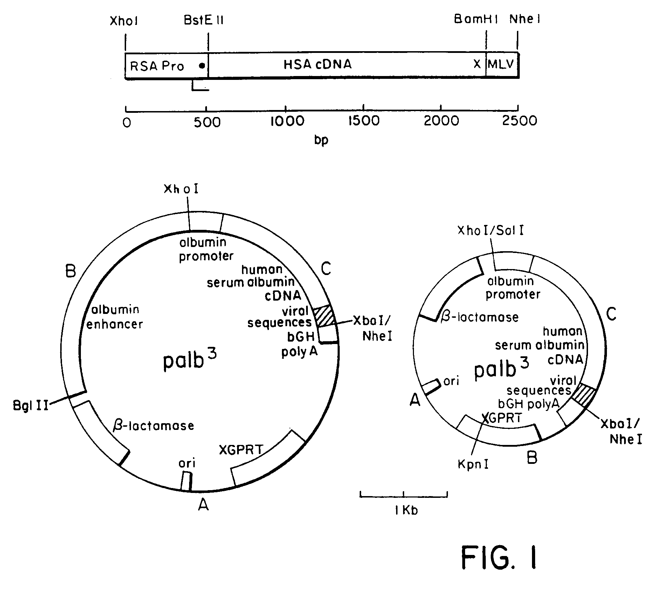

The structures of the relevant portions of palbHSA, palb³ and palb² are shown in figure 1. XGPRT, zanthine-guanine phosphoribosyltransferase; MLV, Moloney murine leukemia virus; RSAPro, rat albumin promoter; HSA cDNA, human serum albumin cDNA; solid circle, translational start site; x, translational termination site.

The plasmid, palb³, is a eukaryotic expression vector that expresses human serum albumin cDNA sequences driven by the rat albumin promoter and the mouse albumin enhancer regions (Figure 1). This vector was constructed in a single three-part ligation with fragments that were cloned in a directional manner. Fragment A: an XbaI to BglII fragment (3.7 kb) of plasmid MTEV.JT, the relevant sequences of which were derived from a precursor described by Pfarr, D.S. et al. DNA 4:461-467 (1988), contains a 231 bp fragment of genomic DNA spanning the polyadenylation signal of the bovine growth hormone gene, β-lactamase and the prokaryotic origin of replication from PUC 19, and a eukaryotic transcriptional unit expressing xanthine-guanine phosphoribosyltransferase (XGPRT). Fragment B: sequences spanning an enhancer located 5' to the mouse albumin gene (-12 to -9 kb) were excised from a pBR322 subclone of a recombinant lambda phage isolated from a mouse genomic library. Gorin, M.B. et al. J. Biol. Chem. 256:1954-1959 (1981). The enhancer elements were removed on an EcoRV to BglII fragment in which the EcoRV site was converted to an XhoI site with synthetic linkers. Fragment C was removed from a previously undescribed retroviral vector, palbHSA, as an XhoI to NheI fragment (2405 bp) which contains the following sequences: genomic DNA of the rat albumin gene from the XbaI site at nucleotide -443 (converted to an XhoI site) to the BstEII site at nucleotide +45 (Urano, Y. et al. J. Biol. Chem. 261:3244-3251 (1986)); cDNA sequences of human serum albumin from the BstEII site at nucleotide +50 to the HindIII site at nucleotide +1787 (converted to a BamHI site) (Urano, et al., supra) and 3' flanking sequences of the Moloney murine leukemia virus from the ClaI site at nucleotide 7674 (converted to a BamHI site) to the NheI site at nucleotide 7846 (Van Beveren, C., Coffin, J., and Hughes, S. in RNA Tumor Viruses, Weiss, R., Teich, N., Varmus, H., and Coffin, J., eds., Cold Spring Harbor Laboratory, Cold Spring Harbor, NY 2nd ed. pp. 766-783 (1985)).

A control vector, palb², lacking the albumin enhancer was constructed (Figure 1) by a single three-part ligation as described above. Fragment A: an XbaI to KpnI fragment of plasmid MTEV.JT (2876 bp) containing the β-lactamase gene and the prokaryotic origin of replication from PUC 19 and a portion of a eukaryotic transcriptional unit expressing XGPRT. Fragment B: a KpnI to SalI fragment of plasmid MTEV.JT (780 bp) containing the rest of the XGPRT transcriptional unit. Fragment C: an XhoI to NheI fragment (2405 bp) of palbHSA described above. Because the enhancer regions are required for high level expression by the albumin promoter (Pinckert, C.A. et al. Genes and Development 1:268-276 (1987)) the palb² plasmid served to control the nonspecific effects of plasmid DNA.

The vectors were cloned in E. coli and purified as described previously (Birnboim, H.C., and Doly, J. Nucleic Acids Res. 7:1513-1518 (1979)). Purity was checked by electrophoresis through agarose gels stained with ethidium bromide (Maniatis, T., Fritsch, E.F., and Sambrook, G. in Molecular Cloning, A Laboratory Manual, Fritsch, E.G. and Maniatis, T., eds., Cold Spring Harbor Laboratory, Cold Spring Harbor, NY pp. 150-161 (1982)).

### The Targetable DNA Carrier

Asialoorosomucoid, prepared from pooled human serum (Wu, G.Y. and Wu, C.H. J. Biol. Chem. 263:14621-14624 (1988); Whitehead, D.H. and Sammons, H.G. Biochim. Biochys. Acta 124:209-211 (1966)), was coupled to poly-L-lysine (Sigma Chemical Co., St. Louis, MO), Mr = 3,800, as described previously using a water soluble carbodiimide (Jung, G. et al. Biochem. Biochys. Res. Commun. 101:599-606 (1981)). In brief, asialoorosomucoid was treated with a 7-fold molar excess of poly-L-lysine at pH 7.4 using 1-ethyl-3-(3-dimethylaminopropyl) carbodiimide (Pierce Chemical Co., Rockford, IL) present in a 154-fold molar excess over poly-L-lysine. After 24 hrs, the conjugate product was purified by gel filtration chromatography and titrated with plasmid DNA using a gel retardation assay as described previously (Wu, G.Y. and Wu, C.H. J. Biol. Chem. 262:4429--4432 (1987)). The optimal ratio of conjugate to DNA for palb³ was determined to be 2.5:1, and for palb², 2.0:1. These ratios were used for all subsequent experiments. The complexed DNA was filtered through 0.45 µ membranes (Millipore Co., Bedford, MA) prior to injection.

### Targeted Gene Delivery

Groups of rats, 2 each, were anesthetized with ketamine-xylazine and then injected intravenously via a tail vein with complexed palb³ DNA, palb² DNA, 500 µg/ml in sterile saline, or saline alone. Fifteen minutes later, the rats were subjected to 66% partial hepatectomy (Wayforth, H.B., in Experimental and Surgical Techniques in the Rat, Academic Press, NY (1980)). At various intervals, blood was drawn, rats were killed, and livers removed and homogenized. DNA was isolated by phenol-chloroform extraction (Blin, N. and Stafford, D.W. Nucl. Acids. Res. 3:2303-2308 (1976)).

### Analysis of Targeted DNA

The quantity and state of human albumin DNA sequences were determined by Southern blot analysis (Southern, E.M. J. Mol. Biol. 98:503-517 (1975)). Liver DNA was isolated two weeks after injection with targeted DNA. Total cellular DNA was isolated and treated with BamHI, XhoI or NruI. Bands were detected by hybridization with ³²P-labeled probes derived from: 1) plasmid MTEV.JT, a 2307 bp EcoRI to BamHI fragment spanning the β-lactamase gene, or 2) the 3'-region of human serum albumin cDNA (1083 bp, BglII to BamHI fragment).

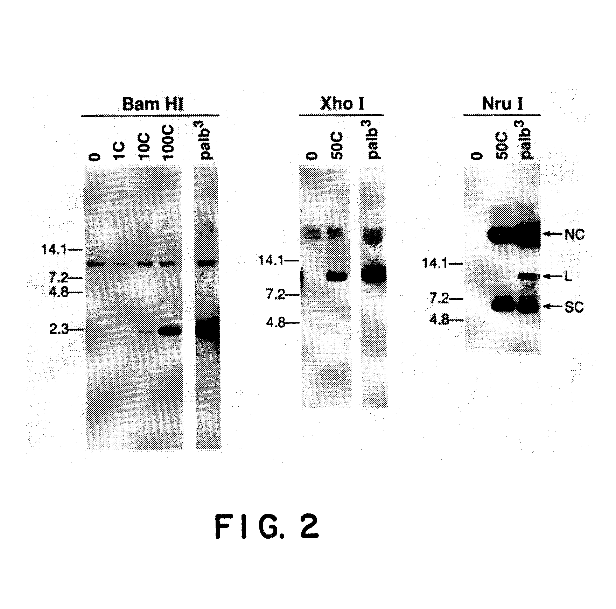

Figure 2 shows representative autoradiographs of DNA blots of liver DNA from Nagase analbuminemic rats 2 weeks after injection with targeted palb³ DNA followed by partial hepatectomy. BamHI DNA from untransfected Nagase rat liver (10 µg) was supplemented with palb³ plasmid DNA as follows: lane "0" contains no plasmid; lane "1C" contains 1 copy (7.5 pg plasmid), lane "10C" contains 10 copies (75 pg plasmid), and lane "100C" contains 100 copies of plasmid/diploid genome (750 pg plasmid). XhoI and NruI DNA from untransfected Nagase rat liver (10 µg) was analyzed alone, lane "0", or in the presence of 50 copies of plasmid/diploid genome (375 pg plasmid), lane "50C". DNA from liver harvested 2 weeks after injection of complexed palb³ DNA was analyzed in lane "palb³". BamHI and XhoI digested DNA blots were hybridized with an albumin cDNA probe; NruI digests were hybridized with the non-albumin-containing plasmid probe MTEV.JT, a 2307 bp EcoRI to BamHI fragment spanning the β-lactamase gene. Molecular size standards are indicated in kilobases along the right borders. (MC = nicked circular, L = linear, and SC = supercoiled DNA).

Restriction of total cellular DNA with BamHI releases the human albumin gene insert from the palb³ plasmid on a 2100 bp fragment. As expected from the increasing amount of standard palb³ added, lanes "1C", "10C" and "100C", show a proportional increase in hybridization of the band at approximately 2.1 kb, the size of the insert (left gel Figure 2). Another band found at approximately 9 kb, likely due to cross-hybridization to endogenous rat sequences (because it was also present in samples from untreated rats as shown in lane "0"), was used as an internal standard for the amount of cellular DNA present in each sample. No band corresponding in size to the insert was found in DNA from untreated rats, lane "0". However, rats treated with palb³, lane "palb³", showed a strong signal at the position expected for the insert, which upon quantitation revealed an average copy number of 1000 copies of the plasmid/diploid genome. Bands larger than the albumin insert were not detected, indicating that no significant rearrangements of the albumin structural gene had occurred.

To characterize the molecular state of the plasmid DNA in palb³-treated liver samples 2 weeks post-injection and partial hepatectomy, total cellular DNA digested with XhoI, which has a single cutting site in the plasmid, and hybridized with the albumin cDNA probe. Figure 2, middle gel, lane "palb³", shows that digestion of palb³-treated liver DNA produced a band that corresponded in size to linearized plasmid. Hybridization to some endogenous rat sequences was also seen in the form of bands greater than 14 kb in size.

To confirm that DNA bands corresponding in size to plasmid were indeed of plasmid origin, total cellular DNA from livers from the palb³-treated rats were digested with NruI which lacks any restriction sites in the plasmid. Samples were probed with a fragment of the plasmid MTEV.JT, spanning the β-lactamase gene but lacking any albumin sequences. This showed two predominant bands corresponding to nicked circular and supercoiled forms of the plasmid. A small band was also seen, corresponding to linearized plasmid. These data indicate that the overwhelmingly predominant portion of retained DNA in liver in these experiments existed as unintegrated circular plasmid DNA. However, because of the presence of hybridizable high molecular weight DNA, the possibility of integration of some plasmid DNA into the host genome cannot be excluded. Rats treated with the enhancerless palb² plasmid showed similar patterns.

### Analysis of Human Albumin mRNA: RNA Dot-Blots

In order to determine whether the targeted, complexed DNA was transcribed, analbuminemic rat livers were assayed by dot blots for the presence of human serum albumin mRNA two weeks after injection and partial hepatectomy. A representative dot blot of RNA extracted from Nagase analbuminemic rat livers from animals 2 weeks after treatment with targeted plasmid DNA or controls followed by partial hepatectomy.

Total RNA was extracted from liver tissue by the method of Chomczynski et al. (Chomczynski, P. and Sacci, N. Anal. Biochem. 162:156-159 (1987)). Serial (1:2) dilutions of RNA starting at 30 µg with or without pretreatment with DNase-free RNase were applied onto a nitrocellulose filter and hybridized to a ³²P-labeled 19-mer synthetic cDNA specific for a human albumin sequence (complementary to sequences of albumin message corresponding to the 695-715 base pair region of human albumin cDNA). Sambrook, J., Fritsch, E.F. and Maniatis, T., eds. Cold Spring Harbor, New York pp. 7.35-7.55 (1989). Row 1, analbuminemic rats treated with saline; row 2, analbuminemic rats treated with palb² plasmid DNA as a targetable complex; row 3, analbuminemic rats treated with palb³ as a targetable complex; row 4, same as row 3 except that the sample was digested with DNase-free RNase prior to hybridization; row 5, RNA from normal Sprague-Dawley rats. NAR, Nagase analbuminemic rats.

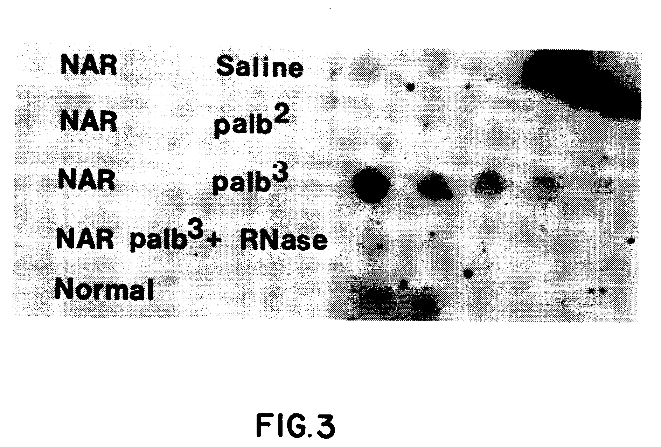

As shown in Figure 3, total RNA from livers of rats that received saline alone, top row; as well as rats that received the enhancerless control plasmid, palb², second row, did not hybridize with the human albumin specific cDNA probe. However, the third row shows that RNA from rats that received the palb³ did produce a strong signal. The fourth row (in which a sample from row 3 was digested with DNase-free RNase prior to hybridization) shows that DNase-free RNase completely abolished the hybridization seen previously in row 3, supporting the conclusion that the signal was due to the presence of RNA. The last row shows that RNA from liver of a normal untreated Sprague-Dawley rat did not hybridize with the probe, indicating that the signal detected in row 3 was not due to hybridization to endogenous rat sequences.

### Analysis of Human Albumin mRNA: RNase Protection Assays

Further evidence for the presence of vector-derived human serum albumin mRNA in liver tissue was provided by RNase protection analysis using a vector-specific RNA probe followed by partial hepatectomy.

RNA was extracted from liver tissue and analyzed by RNase protection assays (Melton, D.A. et al. Nucleic Acids Res. 12:7035-7056 (1984)) using a vector-specific probe. The RNA probe, 3Z-env, complementary to Moloney retrovirus-derived sequences in the 3' untranslated region of the recombinant human albumin transcript was synthesized in vitro as described previously (Wilson, J.M. et al. Proc. Natl. Acad. Sci. 87:8437-8441 (1990)) by cloning this region between the BamHI and XbaI sites of pGEM-3Z(f+), and labeling with ³²P.

RNA from a previously transfected NIH 3T3 cell line that expresses a transcript containing the vector-derived sequence, and RNA from the untransfected NIH 3T3 cells were used as positive and negative controls, respectively. Total cellular RNA from liver tissue was extracted as described above, and 100 µg each were analyzed by RNase protection according to the method of Melton et al. (supra). Lane "3T3" contains RNA (200 ng) from an NIH 3T3 cell line that was made to express a transcript which possesses the vector-derived sequence. A 172 bp fragment that is resistant to RNase A was found at the expected location indicated by the arrow. Lane "palb²" contains RNA (100 µg) from analbuminemic rat liver harvested 2 weeks after transfection with palb²; and lane "palb³", RNA (100 µg) from analbuminemic rat liver harvested 2 weeks after transfection with palb³. Molecular size markers are present in the lane farthest to the right.

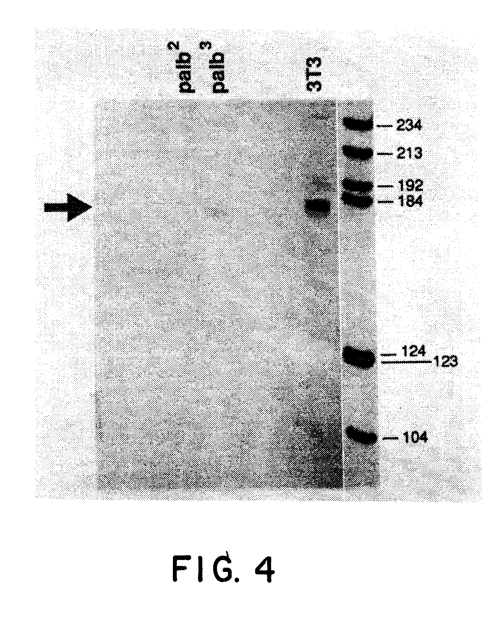

Hybridization of the probe to RNA from NIH 3T3 cells made to express the transcript containing vector sequences (positive control cells), produced a band of the expected size, 172 bp (arrow) that was resistant to digestion with RNase A as shown in Figure 4, lane "3T3". Analysis of RNA from liver harvested 2 weeks after transfection of analbuminemic rats with palb³ DNA complex followed by partial hepatectomy also resulted in a protected band of the expected size (172 bp). Some higher size bands were also present, likely due to incomplete digestion of the hybrid with RNase. However, liver from analbuminemic rats harvested 2 weeks after transfection and partial hepatectomy using the same molar quantities of complexed palb² DNA as in the palb³ DNA experiments, Figure 4, lane "palb²" failed to generate any protected sequences under identical conditions. Similarly, RNA from untransfected NIH 3T3 cells, and untransfected Nagase analbuminemic rats did not produce protected sequences indicating that the observed 172 bp band obtained after palb³ DNA transfection was not due to non-specific hybridization to other endogenous, non-vector-derived RNA sequences. Using RNase protection analysis with probes to endogenous rat albumin and recombinant human albumin on RNA, the level of human albumin mRNA in transfected analbuminemic rat liver was estimated to be between 0.01% and 0.1% of rat albumin mRNA in normal rats (data not shown).

### Assay for Circulating Human Serum Albumin

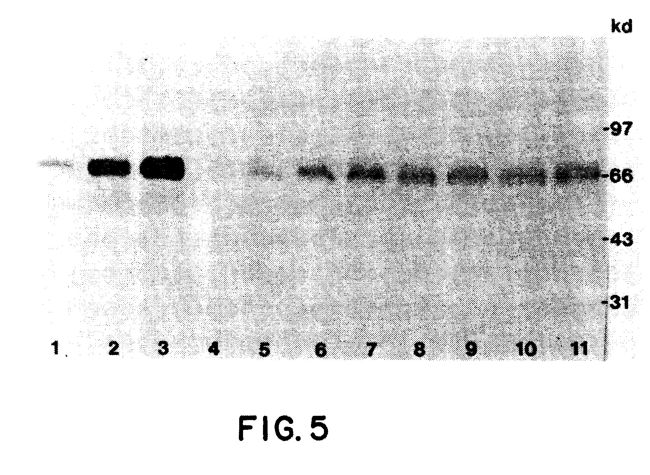

Identification and quantitation of human serum albumin was accomplished by Western blots (Burnette, W.N. Anal. Biochem. 112:195-203 (1981)), using an affinity-purified rabbit anti-human albumin antibody. Figure 5 is a representative Western blot of rat serum samples taken two weeks after treatment of analbuminemic rats with targeted palb³ DNA followed by partial hepatectomy. Serum or standard albumins were applied on a polyacrylamide gel electrophoresis, then transferred to nitrocellulose and exposed to the specific rabbit anti-human albumin antibody. Subsequently the gels were incubated with goat anti-rabbit IgG conjugated to alkaline phosphatase and developed by exposure to BCIP/NBT.

Specifically, 10 µg of human serum albumin, 10 µg rat serum albumin, and 4 µl each of serum from normal rats, untreated analbuminemic rats, and treated analbuminemic rats were applied onto a 10% SDS-polyacrylamide gel (Laemmli, U.K. Nature 227:680-685 (1970)) and run at 150 V for 4.5 hours. Human serum albumin, 20 µg, is shown in lane 1; standard rat serum albumin, 20 µg, lane 2; human albumin, 20 µg, in 4 µl untreated analbuminemic rat serum, lane 3; and 4 µl of serum from: untreated analbuminemic rats, lane 4; normal Sprague-Dawley rats, lane 5; serum from analbuminemic rats treated with palb³ DNA complex, lane 6; analbuminemic rats treated with saline alone, lane 7; analbuminemic rats treated with palb² DNA complex, lane 8.

The gel was electrophoretically transferred onto nitrocellulose using a Trans-Blot cell (Bio-Rad), quenched with blotto (10% powdered non-fat milk in PBS), exposed to anti-human albumin antibody, and then incubated with anti-rabbit IgG conjugated to alkaline phosphatase. The filters were then washed, and developed with BCIP/NBT (Kirkegaard and Perry Lab. Inc.)

Figure 5, lanes 1-5 demonstrate the specificity of the anti-human serum albumin antibody for human albumin; a single band was detected in the blot of standard human albumin, whereas no staining was detected with an equal amount of standard rat serum albumin, lane 2. Albumin is known to bind a number of serum components. To determine whether binding of rat serum components could alter the electrophoretic mobility of human albumin, standard human albumin was mixed with serum from untreated analbuminemic rats. Lane 3 shows that this had no significant effect as the migration position of human albumin remained unchanged. A band at approximately 130 kDa is likely due to the presence of albumin dimers.

The specificity of the anti-human albumin antibody was further demonstrated by the lack of any reaction to either normal rat serum, lane 4; or untreated analbuminemic rat serum, lane 5. However, analbuminemic rats that received the palb³ DNA complex did produce a band corresponding in size to albumin. The level of this circulating human serum albumin was quantitated to be approximately 30 µg/ml, two weeks after injection, lane 6. Control animals that received saline alone, lane 7, or the palb² enhancerless plasmid, lane 8, did not produce detectable human albumin under identical conditions.

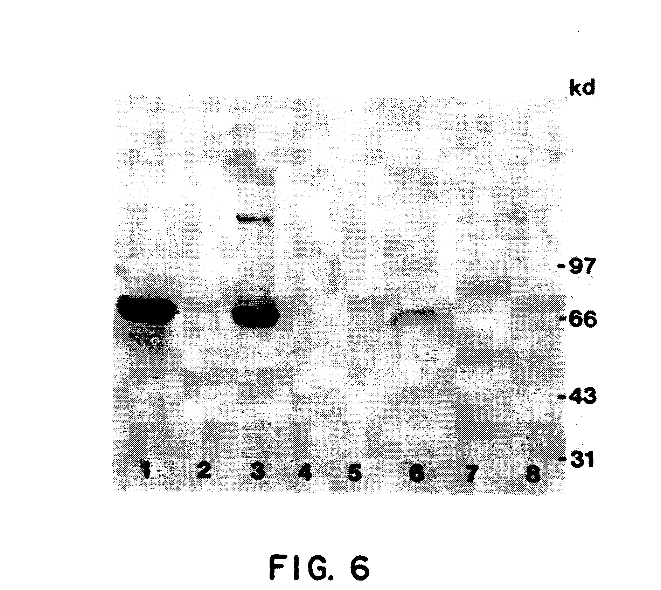

A time course of the appearance of human albumin in the circulation is shown in Figure 6. Rats were treated with palb³ DNA complex followed by partial hepatectomy. At regular intervals, serum was obtained and levels of circulating human serum albumin determined by Western blots as described for Figure 4. Lanes 1-3 contain standard human albumin, 0.1, 1.0 and 10 µg. Lanes 4-11 contain 4 µl serum from treated rats 24 h, 48 h, 72 h, 96 h, 1 week, 2 weeks, 3 weeks, and 4 weeks after injection, respectively. Serum samples or standard human albumin were applied on a polyacrylamide gel electrophoresis, then transferred to nitrocellulose and exposed to a specific rabbit anti-human albumin antibody. Filters were washed and then incubated with goat anti-rabbit IgG conjugated to alkaline phosphatase and developed by exposure to BCIP/NBT.

Serum from a representative analbuminemic rat treated with palb³ DNA complex, lane 4, did not have detectable circulating albumin after 24 hours. However, human albumin was detectable in serum from palb³ DNA-treated analbuminemic rats by 48 hours, lane 5, at a level of approximately 0.05 µg/ml. The level of human albumin rose with time reaching a plateau of 34 µg/ml by the 2nd week, lane 8, and remained at this level without significant change through the 4th week post-injection, lane 11. Using an ELISA method, no anti-human albumin antibodies were detected, at least through the 4th week after transfection (data not shown).

### Example 2

An asialoglycoprotein-polycation conjugate consisting of asialoorosmucoid coupled to poly-L-lysine, was used to form a soluble DNA complex capable of specifically targeting hepatocytes via asialoglycoprotein receptors present on these cells. The DNA comprised a plasmid containing the gene for hepatitis B virus surface antigen.

### Expression Vector Containing Gene Encoding Hepatitis B Virus Surface Antigen

Plasmid pSVHBVs was obtained from Dr. T. Jake Liang (Massachusetts General Hospital, Boston, MA). The plasmid (approximately 3.6kbp) is a pUC derivative containing the SV40 origin of replication and the open reading frame for hepatitis B surface antigen (as part of a 1984 bp insert) driven by the SV40 promoter. The plasmid was cloned and purified as described above.

### The Targetable DNA Carrier

Asialoorosmucoid (ASOR) was prepared as described above. ASOR was coupled to poly-L-lysine (Sigma Chemical Co., St. Louis, MO) Mr = 59,000 (7:1 molar ratio) via disulfide bonds using N-succinimidyl 3-(2-pyridyldithio) propronate (SPDP) to form the labeled conjugate. ASOR was also coupled to poly-L-lysine Mr = 41,100 (1:1 molar ratio) at pH 7.4 using 1-ethyl-3-(3-dimethyl-aminopropyl) carbodiimide (Pierce Chemical Co., Rockford, IL).

The conjugates were purified by cation exchange chromatography using a high pressure liquid chromatographic system (Rainan) employing an Aquapore C-300 column (Rainan) and stepwise elution with 0.1 M sodium acetate pH 5.0, 2.5, 2.25 and 2.0. The second peak eluted from the column as detected by U.V. absorption at 230 nm was determined as the optimal conjugate (Jung, G. et al. Biochem Biochys. Res. Commun. 101:599-606 (1981)).

The optimal proportion of DNA to mix with the conjugate to form a soluble complex was determined using gel retardation assay described above. Samples containing equal amounts of DNA in .15 M NaCl were mixed with increasing amounts of the conjugate in .15 M NaCl to determine the conjugate to DNA molar ratio which completely retards DNA migration in the gel. The amount of conjugate needed to bind 50-75% of the DNA was calculated and used to form the molecular complex (in order to ensure solubility of the complex). To form the soluble molecular complex, the conjugate solution was added very slowly to the DNA solution by a peristaltic pump at a speed of 0.1 ml/min with constant mixing. An aliquot was taken and absorbance at A₂₆₀ₙₘ was determined to monitor the amount of DNA. Another aliquot was taken and run on an agarose gel to verify the formation of complex. The solution containing the complex was filtered though a 0.45 µ membrane filter and washed with saline. Aliquots were taken for testing as above.

### Targeted Gene Delivery

Groups of 150 g female rats (Sprague-Dawley), 2 each were anesthetized with hetamine-zylazine and then injected very slowly intravenously via the tail vein. Rats in one group received the conjugate prepared with poly-L-lysine Mr = 59,000 using SPDP coupling and complexed with 5 mg DNA. The other group of rats received the conjugate prepared with poly-L-lysine Mr = 41,100 using carbodiimide coupling and complexed with 1.4 mg DNA. At 24 hour intervals, the rats were bled and serum was be obtained for assay of hepatitis-B virus surface antigen. (Auszyme Monoclonal, EIA Kit for detection of HBV - Abbott). The resultant solution color change was measured at A^{492nm} for 200 µl of serum. The results are shown in Table 1.

**Table 1**

| Results are given as optical density units at A^{492nm} for 200 µl serum Time (Days) | | | | | | | | | | | |
|---|---|---|---|---|---|---|---|---|---|---|---|
| | 0 | 1 | 2 | 3 | 4 | 6 | 7 | 8 | 14 | 30 | 60 |
| Rat | | | | | | | | | | | |
| 1 | .012 | .024 | .261 | .33 | .23 | | | | | | |
| 2 | .011 | .048 | .137 | .28 | .25 | .22 | .10 | .09 | .175 | .33 | .15 |
| 3 | .016 | .26 | .22 | .08 | | | | | | | |
| 4 | .018 | .22 | .24 | .16 | | | | | | | |

The expression HBV surface antigen detected for the rats that received the soluble molecular complex consisting of the conjugate prepared via SPDP coupling and 5 mg DNA (rats #1 & #2) persisted for at least 4 days and increased consistently reaching a maximum of .33. The expression detected for the rats that received the soluble molecular complex consisting of the conjugate prepared via carbodiimide coupling and 1.4 mg DNA (rats 13 & #4) also persisted for at least 3 days and increased consistently reaching a maximum of approximately .25.

### Equivalents

Those skilled in the art will recognize, or be able to ascertain using no more than routine experimentation, numerous equivalents to the specific procedures described herein. Such equivalents are considered to be within the scope of this invention and are covered by the following claims.

## Claims (Claims for the following Contracting State(s): AT, BE, CH, LI, DE, DK, FR, GB, IT, LU, MC, NL, SE)

1. A soluble molecular complex for targeting a gene encoding a secretory protein to a specific cell, the complex comprising an expressible gene encoding the secretory protein in a form suitable for expression, processing and secretion of the protein by the target cell, the gene being complexed with a carrier comprising a cell-specific binding agent and a gene-binding agent which complexes the gene under extracellular condition and releases the gene under intracellular conditions in expressible form.

2. A soluble molecular complex of claim 1, wherein the expressible gene is a DNA molecule.

3. A soluble molecular complex of claims 1 or 2, wherein the expressible gene encodes albumin.

4. A soluble molecular complex of claims 1 or 2, wherein the expressible gene encodes a blood coagulation factor.

5. A soluble molecular complex of claim 4, wherein the blood coagulation factor is selected from the group consisting of factor V, VII, VIII, IX, X or XI.

6. A soluble molecular complex of claims 1 to 5, wherein the gene-binding agent is a polycation.

7. A soluble molecular complex of claim 6, wherein the polycation is polylysine.

8. A soluble molecular complex of claims 1 to 7, wherein the cell-specific binding agent binds a surface receptor of the cell which mediates endocytosis.

9. A soluble molecular complex of claims 1 to 8, wherein the cell-specific binding agent is a ligand for an asialoglycoprotein receptor.

10. A soluble molecular complex of claim 9, wherein the ligand is an asialoglycoprotein and the targeted cell is a hepatocyte.

11. A soluble molecular complex of claims 1 to 10, wherein the targeted cell is a hepatocyte, the cell-specific binding agent is a ligand for the asialoglycoprotein receptor and the gene-binding agent is a polycation.

12. A soluble molecular complex of claim 11, wherein the expressible gene encodes the factor VIII or factor IX protein.

13. A soluble molecular complex of claims 1 to 12, wherein the expressible gene is complexed with the gene-binding agent by a noncovalent bond.

14. A soluble molecular complex of claims 1 to 13, wherein the expressible gene is complexed with the gene-binding agent so that the gene is released in functional form under intracellular conditions.

15. A soluble molecular complex of claims 1 to 14, wherein the gene is contained in an expression vector along with genetic regulatory elements necessary for expression of the gene and secretion of a gene-encoded product by the hepatocyte.

16. A soluble molecular complex of claim 15, wherein the expression vector is a plasmid or viral DNA.

17. A soluble molecular complex of claims 1 to 16, wherein the ratio of carrier to gene ranges from 1:5 to 5:1.

18. A pharmaceutical composition comprising a solution of the molecular complex of claims 1 to 17 and a physiologically acceptable vehicle.

19. Use of a soluble molecular complex of claims 1 to 17 for the preparation of a pharmaceutical composition for delivering an expressible gene encoding a secretory protein to a specific cell of an organism for expression and secretion of the gene-encoded product by the cell.

20. Use of a soluble molecular complex of claims 1 to 17 for the preparation of a pharmaceutical composition for transfecting hepatocytes in vivo with a gene encoding a secretory protein.

21. Use of claims 19 or 20, wherein the pharmaceutical composition is for intravenous administration.

22. Use of claims 19 to 21, wherein the hepatocytes are transfected to correct or alleviate an inherited or acquired abnormality in an organism.

## Claims (Claims for the following Contracting State(s): ES, GR)

1. A method for the preparation of a soluble molecular complex for targeting a gene encoding a secretory protein to a specific cell, characterized in that an expressible gene encoding the secretory protein in a form suitable for expression, processing and secretion of the protein by the target cell is complexed with a carrier comprising a cell-specific binding agent and a gene-binding agent which complexes the gene under extracellular condition and releases the gene under intracellular conditions in expressible form.

2. The method of claim 1, wherein the expressible gene is a DNA molecule.

3. The method of claims 1 or 2, wherein the expressible gene encodes albumin.

4. The method of claims 1 or 2, wherein the expressible gene encodes a blood coagulation factor.

5. The method of claim 4, wherein the blood coagulation factor is selected from the group consisting of factor V, VII, VIII, IX, X or XI.

6. The method of claims 1 to 5, wherein the gene-binding agent is a polycation.

7. The method of claim 6, wherein the polycation is polylysine.

8. The method of claims 1 to 7, wherein the cell-specific binding agent binds a surface receptor of the cell which mediates endocytosis.

9. The method of claims 1 to 8, wherein the cell-specific binding agent is a ligand for an asialoglycoprotein receptor.

10. The method of claim 9, wherein the ligand is an asialoglycoprotein and the targeted cell is a hepatocyte.

11. The method of claims 1 to 10, wherein the targeted cell is a hepatocyte, the cell-specific binding agent is a ligand for the asialoglycoprotein receptor and the gene-binding agent is a polycation.

12. The method of claim 11, wherein the expressible gene encodes the factor VIII or factor IX protein.

13. The method of claims 1 to 12, wherein the expressible gene is complexed with the gene-binding agent by a noncovalent bond.

14. The method of claims 1 to 13, wherein the expressible gene is complexed with the gene-binding agent so that the gene is released in functional form under intracellular conditions.

15. The method of claims 1 to 14, wherein the gene is contained in an expression vector along with genetic regulatory elements necessary for expression of the gene and secretion of a gene-encoded product by the hepatocyte.

16. The method of claim 15, wherein the expression vector is a plasmid or viral DNA.

17. The method of claims 1 to 16, wherein the ratio of carrier to gene ranges from 1:5 to 5:1.

18. A method for the preparation of a pharmaceutical composition comprising a molecular complex for targeting a gene encoding a secretory protein to a specific cell, characterized in that a soluble molecular complex obtainable by the method of claims 1 to 17 is dissolved in a physiologically acceptable vehicle.

19. Use of a soluble molecular complex obtainable by the method of claims 1 to 17 for the preparation of a pharmaceutical composition for delivering an expressible gene encoding a secretory protein to a specific cell of an organism for expression and secretion of the gene-encoded product by the cell.

20. Use of a soluble molecular complex obtainable by the method of claims 1 to 17 for the preparation of a pharmaceutical composition for transfecting hepatocytes in vivo with a gene encoding a secretory protein.

21. Use of claims 19 or 20, wherein the pharmaceutical composition is for intravenous administration.

22. Use of claims 19 to 21, wherein the hepatocytes are transfected to correct or alleviate an inherited or acquired abnormality in an organism.

## Patentansprüche (Patentansprüche für folgende(n) Vertragsstaat(en): AT, BE, CH, LI, DE, DK, FR, GB, IT, LU, MC, NL, SE)

1. Löslicher Molekülkomplex zum Dirigieren (Targeting) eines ein sekretorisches Protein kodierenden Gens zu einer spefizischen Zelle, wobei der Komplex ein das sekretorische Protein kodierendes exprimierbares Gen in einer Form umfaßt, die für Expression, Processing und Sekretion des Proteins durch die Zielzelle geeignet ist, wobei das Gen mit einem Träger komplexiert ist, der ein zellspezifisches Bindungsmittel und ein Gen-bindendes Mittel, das das Gen unter extrazellulären Bedingungen komplexiert und das Gen unter intrazellulären Bedingungen in exprimierbarer Form freisetzt, umfaßt.

2. Löslicher Molekülkomplex nach Anspruch 1, bei dem das exprimierbare Gen ein DNA-Molekül ist.

3. Löslicher Molekülkomplex nach den Ansprüchen 1 oder 2, bei dem das exprimierbare Gen Albumin kodiert.

4. Löslicher Molekülkomplex nach den Ansprüchen 1 oder 2, bei dem das exprimierbare Gen einen Blutgerinnungsfaktor kodiert.

5. Löslicher Molekülkomplex nach Anspruch 4, bei dem der Blutgerinnungsfaktor aus der Gruppe, bestehend aus Faktor V, VII, VIII, IX, X oder XI, ausgewählt ist.

6. Löslicher Molekülkomplex nach den Ansprüchen 1 bis 5, bei dem das Gen-bindende Mittel ein Polykation ist.

7. Löslicher Molekülkomplex nach Anspruch 6, bei dem das Polykation Polylysin ist.

8. Löslicher Molekülkomplex nach den Ansprüchen 1 bis 7, bei dem das zellspezifische Bindungsmittel einen Oberflächenrezeptor der Zelle bindet, der Endozytose vermittelt.

9. Löslicher Molekülkomplex nach den Ansprüchen 1 bis 8, bei dem das zellspezifische Bindungsmittel ein Ligand für einen Asialoglykoproteinrezeptor ist.

10. Löslicher Molekülkomplex nach Anspruch 9, bei dem der Ligand ein Asialoglykoprotein ist und die Zelle, auf die das Targeting gerichtet ist, ein Hepatozyt ist.

11. Löslicher Molekülkomplex nach den Ansprüchen 1 bis 10, bei dem die Zelle, auf die das Targeting gerichtet ist, ein Hepatozyt ist, das zellspezifische Bindungsmittel ein Ligand für den Asialoglykoproteinrezeptor ist und das Gen-bindende Mittel ein Polykation ist.

12. Löslicher Molekülkomplex nach Anspruch 11, bei dem das exprimierbare Gen das Faktor VIII- oder Faktor IX-Protein kodiert.

13. Löslicher Molekülkomplex nach den Ansprüchen 1 bis 12, bei dem das exprimierbare Gen mit dem Gen-bindenden Mittel durch eine nicht-kovalente Bindung komplexiert ist.

14. Löslicher Molekülkomplex nach den Ansprüchen 1 bis 13, bei dem das exprimierbare Gen mit dem Gen-bindenden Mittel so komplexiert ist, daß das Gen unter intrazellulären Bedingungen in funktionsfähiger Form freigesetzt wird.

15. Löslicher Molekülkomplex nach den Ansprüchen 1 bis 14, bei dem das Gen in einem Expressionsvektor zusammen mit genetischen Regulationselementen enthalten ist, die für eine Expression des Gens und eine Sekretion eines durch das Gen kodierten Produkts durch den Hepatozyten erforderlich sind.

16. Löslicher Molekülkomplex nach Anspruch 15, bei dem der Expressionsvektor ein Plasmid oder virale DNA ist.

17. Löslicher Molekülkomplex nach den Ansprüchen 1 bis 16, bei dem das Verhältnis von Träger zu Gen im Bereich von 1:5 bis 5:1 liegt.

18. Pharmazeutische Zusammensetzung, die eine Lösung des Molekülkomplexes nach den Ansprüchen 1 bis 17 und eines physiologisch akzeptablen Vehikels umfaßt.

19. Verwendung eines löslichen Molekülkomplexes nach den Ansprüchen 1 bis 17 für die Herstellung einer pharmazeutischen Zusammensetzung zum Abgeben eines exprimierbaren, ein sekretorisches Protein kodierenden Gens an eine spezifische Zelle eines Organismus zur Expression und Sekretion des durch das Gen kodierten Produkts durch die Zelle.

20. Verwendung eines löslichen Molekülkomplexes nach den Ansprüchen 1 bis 17 für die Herstellung einer pharmazeutischen Zusammensetzung zum Transfizieren von Hepatozyten in vivo mit einem Gen, das ein sekretorisches Protein kodiert.

21. Verwendung nach den Ansprüchen 19 oder 20, bei der die pharmazeutische Zusammensetzung für eine intravenöse Verabreichung bestimmt ist.

22. Verwendung nach den Ansprüchen 19 bis 21, bei der die Hepatozyten transfiziert werden, um eine vererbte oder erworbene Abnormalität in einem Organismus zu korrigieren oder zu lindern.

## Patentansprüche (Patentansprüche für folgende(n) Vertragsstaat(en): ES, GR)

1. Verfahren zur Herstellung eines löslichen Molekülkomplexes zum Diridieren (Targeting) eines ein sekretorisches Protein kodierenden Gens zu einer spezifischen Zelle, dadurch gekennzeichnet, daß man ein das sekretorische Protein kodierendes exprimierbares Gen in einer Form, die für Expression, Processing und Sekretion des Proteins durch die Zielzelle geeignet ist, mit einem Träger komplexiert, der ein zellspezifisches Bindungsmittel und ein Gen-bindendes Mittel, das das Gen unter extrazellulären Bedingungen komplexiert und das Gen unter intrazellulären Bedingungen in exprimierbarer Form freisetzt, umfaßt.

2. Verfahren nach Anspruch 1, bei dem das exprimierbare Gen ein DNA-Molekül ist.

3. Verfahren nach den Ansprüchen 1 oder 2, bei dem das exprimierbare Gen Albumin kodiert.

4. Verfahren nach den Ansprüchen 1 oder 2, bei dem das exprimierbare Gen einen Blutgerinnungsfaktor kodiert.

5. Verfahren nach Anspruch 4, bei dem der Blutgerinnungsfaktor aus der Gruppe, bestehend aus Faktor V, VII, VIII, IX, X oder XI, ausgewählt ist.

6. Verfahren nach den Ansprüchen 1 bis 5, bei dem das Gen-bindende Mittel ein Polykation ist.

7. Verfahren nach Anspruch 6, bei dem das Polykation Polylysin ist.

8. Verfahren nach den Ansprüchen 1 bis 7, bei dem das zellspezifische Bindungsmittel einen Oberflächenrezeptor der Zelle bindet, der Endozytose vermittelt.

9. Verfahren nach den Ansprüchen 1 bis 8, bei dem das zellspezifische Bindungsmittel ein Ligand für einen Asialoglykoproteinrezeptor ist.

10. Verfahren nach Anspruch 9, bei dem der Ligand ein Asialoglykoprotein ist und die Zelle, auf die das Targeting gerichtet ist, ein Hepatozyt ist.

11. Verfahren nach den Ansprüchen 1 bis 10, bei dem die Zelle, auf die das Targeting gerichtet ist, ein Hepatozyt ist, das zellspezifische Bindungsmittel ein Ligand für den Asialoglykoproteinrezeptor ist und das Gen-bindende Mittel ein Polykation ist.

12. Verfahren nach Anspruch 11, bei dem das exprimierbare Gen das Faktor VIII- oder Faktor IX-Protein kodiert.

13. Verfahren nach den Ansprüchen 1 bis 12, bei dem man das exprimierbare Gen mit dem Gen-bindenden Mittel durch eine nicht-kovalente Bindung komplexiert ist.

14. Verfahren nach den Ansprüchen 1 bis 13, bei dem man das exprimierbare Gen mit dem Gen-bindenden Mittel so komplexiert, daß das Gen unter intrazellulären Bedingungen in funktionsfähiger Form freigesetzt wird.

15. Verfahren nach den Ansprüchen 1 bis 14, bei dem das Gen in einem Expressionsvektor zusammen mit genetischen Regulationselementen enthalten ist, die für eine Expression des Gens und eine Sekretion eines durch das Gen kodierten Produkts durch den Hepatozyten erforderlich sind.

16. Verfahren nach Anspruch 15, bei dem der Expressionsvektor ein Plasmid oder virale DNA ist.

17. Verfahren nach den Ansprüchen 1 bis 16, bei dem das Verhältnis von Träger zu Gen im Bereich von 1:5 bis 5:1 liegt.

18. Verfahren zur Herstellung einer pharmazeutischen Zusammensetzung, die einen Molekülkomplex zum Targeting eines ein sekretorisches Protein kodierenden Gens zu einer speziellen Zelle umfaßt, dadurch gekennzeichnet, daß man einen löslichen Molekülkomplex, der durch das Verfahren nach den Ansprüchen 1 bis 17 erhältlich ist, in einem physiologisch akzeptablen Träger löst.

19. Verwendung eines löslichen Molekülkomplexes, der durch das Verfahren nach den Ansprüchen 1 bis 17 erhältlich ist, für die Herstellung einer pharmazeutischen Zusammensetzung zum Abgeben eines exprimierbaren, ein sekretorisches Protein kodierenden Gens an eine spezifische Zelle eines Organismus zur Expression und Sekretion des durch das Gen kodierten Produkts durch die Zelle.

20. Verwendung eines löslichen Molekülkomplexes, der durch das Verfahren nach den Ansprüchen 1 bis 17 erhältlich ist, für die Herstellung einer pharmazeutischen Zusammensetzung zum Transfizieren von Hepatozyten in vivo mit einem Gen, das ein sekretorisches Protein kodiert.

21. Verwendung nach den Ansprüchen 19 oder 20, bei der die pharmazeutische Zusammensetzung für eine intravenöse Verabreichung bestimmt ist.

22. Verwendung nach den Ansprüchen 19 bis 21, bei der die Hepatozyten transfiziert werden, um eine vererbte oder erworbene Abnormalität in einem Organismus zu korrigieren oder zu lindern.

## Revendications (Revendications pour l'(les) Etat(s) contractant(s) suivant(s): AT, BE, CH, LI, DE, DK, FR, GB, IT, LU, MC, NL, SE)

1. Complexe moléculaire soluble destiné à diriger un gène codant pour une protéine de sécrétion vers une cellule spécifique, le complexe comprenant un gène exprimable codant pour la protéine de sécrétion sous une forme appropriée à l'expression, à la maturation et à la sécrétion de la protéine par la cellule cible, le gène étant complexé à un support comprenant un agent de liaison spécifique de la cellule et un agent de liaison au gène qui forme un complexe avec le gène dans les conditions extracellulaires et libère le gène sous une forme exprimable dans des conditions intracellulaires.

2. Complexe moléculaire soluble selon la revendication 1, le gène exprimable étant une molécule d'ADN.

3. Complexe moléculaire soluble selon la revendication 1 ou la revendication 2, dans lequel le gène exprimable code pour l'albumine.

4. Complexe moléculaire soluble selon la revendication 1 ou la revendication 2, dans lequel le gène exprimable code pour un facteur de coagulation du sang.

5. Complexe moléculaire soluble selon la revendication 4, dans lequel le facteur de coagulation du sang est choisi au sein du groupe constitué par les facteurs V, VII, VIII, I, X ou XI.

6. Complexe moléculaire soluble selon l'une des revendications 1 à 5, dans lequel l'agent de liaison au gène est un polycation.

7. Complexe moléculaire soluble selon la revendication 6, dans lequel le polycation est la polylysine.

8. Complexe moléculaire soluble selon l'une des revendications 1 à 7 dans lequel l'agent de liaison spécifique de la cellule se lie à un récepteur de surface de la cellule qui médie l'endocytose.

9. Complexe moléculaire soluble selon l'une des revendications 1 à 8 dans lequel l'agent de liaison spécifique de la cellule est un ligand qui se lie à un récepteur de l'asialoglycoprotéine.

10. Complexe moléculaire soluble selon la revendication 9, dans lequel le ligand est une asialoglycoprotéine et la cellule cible est un hépatocyte.

11. Complexe moléculaire soluble selon l'une des revendications 1 à 10 dans lequel la cellule cible est un hépatocyte, l'agent de liaison spécifique de la cellule est un ligand qui se lie au récepteur de l'asialoglycoprotéine et l'agent de liaison au gène est un polycation.

12. Complexe moléculaire soluble selon la revendication 11, dans lequel le gène exprimable code pour la protéine du facteur VIII ou du facteur IX.

13. Complexe moléculaire soluble selon l'une des revendications 1 à 12 dans lequel le gène exprimable est complexé à l'agent de liaison au gène par une liaison non covalente.

14. Complexe moléculaire soluble selon l'une des revendications 1 à 13 dans lequel le gène exprimable est complexé à l'agent de liaison au gène de telle sorte que le gène soit libéré sous sa forme fonctionnelle dans des conditions intracellulaires.

15. Complexe moléculaire soluble selon l'une des revendications 1 à 14 dans lequel le gène est contenu dans un vecteur d'expression avec les éléments de régulation nécessaires à l'expression du gène et à la sécrétion par les hépatocytes d'un produit codé par le gène.

16. Complexe moléculaire soluble selon la revendication 15, dans lequel le vecteur d'expression est un ADN plasmidique ou viral.

17. Complexe moléculaire soluble selon l'une des revendications 1 à 16 dans lequel le rapport support :gène est compris entre 1 :5 et 5 :1.

18. Composition pharmaceutique comprenant une solution du complexe moléculaire des revendications 1 à 17 et véhicule physiologiquement acceptable.

19. Utilisation d'un complexe moléculaire des revendications 1 à 17 pour la préparation d'une composition pharmaceutique destinée à la délivrance d'un gène exprimable codant pour une protéine de sécrétion à une cellule spécifique d'un organisme pour l'expression et la sécrétion par la cellule du produit codé par le gène.

20. Utilisation d'un complexe moléculaire des revendications 1 à 17 pour la préparation d'une composition pharmaceutique destinée à transfecter des hépatocytes in vivo avec un gène codant pour une protéine de sécrétion.

21. Utilisation des revendications 19 à 20, dans laquelle la composition pharmaceutique est pour administration intraveineuse.

22. Utilisation des revendications 19 à 21, dans laquelle les hépatocytes sont transfectés afin de corriger ou de soulager une anomalie héréditaire ou acquise dans un organisme.

## Revendications (Revendications pour l'(les) Etat(s) contractant(s) suivant(s): ES, GR)

1. Procédé de préparation d'un complexe moléculaire soluble destiné à diriger un gène codant pour une protéine de sécrétion vers une cellule spécifique, le complexe comprenant un gène exprimable codant pour la protéine de sécrétion sous une forme appropriée à l'expression, à la maturation et à la sécrétion de la protéine par la cellule cible, le gène étant complexé à un support comprenant un agent de liaison spécifique de la cellule et un agent de liaison au gène qui forme un complexe avec le gène dans les conditions extracellulaires et libère le gène sous une forme exprimable dans des conditions intracellulaires.

2. Procédé selon la revendication 1, le gène exprimable étant une molécule d'ADN

3. Procédé selon la revendication 1 ou la revendication 2, dans lequel le gène exprimable code pour l'albumine.

4. Procédé selon la revendication 1 ou la revendication 2, dans lequel le gène exprimable code pour un facteur de coagulation du sang.

5. Procédé selon la revendication 4, dans lequel le facteur de coagulation du sang est choisi au sein du groupe constitué par les facteurs V, VII, VIII, I, X ou XI.

6. Procédé selon l'une des revendications 1 à 5, dans lequel l'agent de liaison au gène est un polycation.

7. Procédé selon la revendication 6, dans lequel le polycation est la polylysine.

8. Procédé selon l'une des revendications 1 à 7 dans lequel l'agent de liaison spécifique de la cellule se lie à un récepteur de surface de la cellule qui médie l'endocytose.

9. Procédé selon l'une des revendications 1 à 8 dans lequel l'agent de liaison spécifique de la cellule est un ligand qui se lie à un récepteur de l'asialoglycoprotéine.

10. Procédé selon la revendication 9, dans lequel le ligand est une asialoglycoprotéine et la cellule cible est un hépatocyte.

11. Procédé selon l'une des revendications 1 à 10 dans lequel la cellule cible est un hépatocyte, l'agent de liaison spécifique de la cellule est un ligand qui se lie au récepteur de l'asialoglycoprotéine et l'agent de liaison au gène est un polycation.

12. Procédé selon la revendication 11, dans lequel le gène exprimable code pour la protéine du facteur VIII ou du facteur IX.

13. Procédé selon l'une des revendications 1 à 12 dans lequel le gène exprimable est complexé à l'agent de liaison au gène par une liaison non covalente.

14. Complexe moléculaire soluble selon l'une des revendications 1 à 13 dans lequel le gène exprimable est complexé à l'agent de liaison au gène de telle sorte que le gène soit libéré sous sa forme fonctionnelle dans des conditions intracellulaires.

15. Procédé selon l'une des revendications 1 à 14 dans lequel le gène est contenu dans un vecteur d'expression avec les éléments de régulation nécessaires à l'expression du gène et à la sécrétion par les hépatocytes d'un produit codé par le gène.

16. Procédé soluble selon la revendication 15, dans lequel le vecteur d'expression est un ADN plasmidique ou viral.

17. Procédé selon l'une des revendications 1 à 16 dans lequel le rapport support :gène est compris entre 1 :5 et 5 :1.

18. Procédé de préparation d'une composition pharmaceutique comprenant un complexe moléculaire destiné à diriger un gène codant pour une protéine de sécrétion vers une cellule spécifique, caractérisé en ce que un complexe moléculaire soluble qui peut être obtenu par la méthode des revendications 1 à 17 est dissous dans un véhicule physiologiquement acceptable.

19. Utilisation d'un complexe moléculaire des revendications 1 à 17 pour la préparation d'une composition pharmaceutique destinée à la délivrance d'un gène exprimable codant pour une protéine de sécrétion à une cellule spécifique d'un organisme pour l'expression et la sécrétion par la cellule du produit codé par le gène.

20. Utilisation d'un complexe moléculaire des revendications 1 à 17 pour la préparation d'une composition pharmaceutique destinée à transfecter des hépatocytes in vivo avec un gène codant pour une protéine de sécrétion.

21. Utilisation des revendications 19 à 20, dans laquelle la composition pharmaceutique est pour administration intraveineuse.

22. Utilisation des revendications 19 à 21, dans laquelle les hépatocytes sont transfectés afin de corriger ou de soulager une anomalie héréditaire ou acquise dans un organisme.
